Europäisches Patentamt

European Patent Office

Office européen des brevets

(19)

(11) Veröffentlichungsnummer : **0 021 009**
**B1**

(12) **EUROPÄISCHE PATENTSCHRIFT**

(45) Veröffentlichungstag der Patentschrift :
22.06.83

(21) Anmeldenummer : 80102713.7

(22) Anmeldetag : 16.05.80

(51) Int. Cl.³ : **A 61 K 47/00, A 61 K 9/14,**
**A 61 K 9/18, G 01 N 33/50**

(54) **Verfahren zur Herstellung eines festen Formkörpers mit einem Gehalt an einem Arzneimittelwirkstoff oder einem Diagnostikum.**

(30) Priorität : 21.06.79 DE 2925009

(43) Veröffentlichungstag der Anmeldung :
07.01.81 Patentblatt 81/01

(45) Bekanntmachung des Hinweises auf die Patenterteilung : 22.06.83 Patentblatt 83/25

(84) Benannte Vertragsstaaten :
**AT BE CH DE FR GB IT LI LU NL SE**

(73) Patentinhaber : **BASF Aktiengesellschaft**
**Carl-Bosch-Strasse 38**
**D-6700 Ludwigshafen (DE)**

(72) Erfinder : **Maurer, Robert, Dr.**
**Leininger Strasse 12**
**D-6719 Wattenheim (DE)**

(56) Entgegenhaltungen :
DE A 2 441 761
DE A 2 451 161
DE A 2 834 308
FR A 2 259 836
CHEMICAL ABSTRACTS, Band 83, Nr. 23, 8. Dezember 1975, Seite 80, Zusammenfassung Nr. 188805g, Columbus, Ohio, US, B. VON SYDOW : « Influence of various protectants on the stability of freeze-dried alfalfa mosaic virus and potato virus X »
CHEMICAL ABSTRACTS, Band 90, Nr. 16, 16. April 1979, Seite 372, Zusammenfassung Nr. 127484z, Columbus, Ohio, US, G. STAMPF et al. : « Technology of freeze-drying of rimazolium solutions and examination of the samples »
CHEMICAL ABSTRACTS, Band 88, Nr. 11, 13. März 1978, Seite 95, Zusammenfassung Nr. 69648b, Columbus, Ohio, US, T. VITANOV :

(56) Entgegenhaltungen :
« Studies on the protective properties of varying molecular weight dextran during lyophilization of Escherichia coli »
CHEMICAL ABSTRACTS, Band 83, Nr. 1, 7. Juli 1975, Seite 615, Zusammenfassung Nr. 7106m, Columbus, Ohio, US, A. I. VALAKHANOVICH et al. : « Use of freeze drying for preservation of the commercial strain of Leuconostoc mesenteroides SF-4
CHEMICAL ABSTRACTS, Band 87, Nr. 9, 29. August 1977, Seite 236, Zusammenfassung Nr. 64650r, Columbus, Ohio, US
Schw. Apotheker Zeitung 96 (1958), S. 506-510

Die Akte enthält technische Angaben, die nach dem Eingang der Anmeldung eingereicht wurden und die nicht in dieser Patentschrift enthalten sind.

Verfahren zur Herstellung eines festen Formkörpers mit einem Gehalt an einem Arzneimittelwirkstoff oder einem Diagnostikum

Die Erfindung betrifft ein Verfahren zur Herstellung eines festen Formkörpers mit einem Gehalt an einem Arzneimittelwirkstoff oder einem Diagnostikum.

Substanzen, wie pharmazeutische Wirkstoffe und empfindliche Stoffe wie Enzyme, die als Diagnostika verwendet werden, müssen in der Regel in eine gut verwendbare Form gebracht werden. Hierfür gibt es bereits viele Möglichkeiten, wie Tablettierung, Dragierung, Abfüllen in Steckkapseln u. a. Diese Verfahren bestehen aber in der Regel aus mehreren verschiedenen Stufen und eignen sich in vielen Fällen nicht für hochempfindliche Substanzen.

Es ist weiter bekannt, Wirkstoffe oder Mikroorganismen mit wasserlöslichen hochmolekularen Verbindungen, beispielsweise Dextran, zu stabilisieren (DE-A-2 451 161 ; DE-A-2 834 308 ; C.A. *90*, 127 484 z (1979) ; C.A. *88*, 69 648 b (1978) ; C.A. *87*, 64 650 r (1977) bzw. leichter löslich zu machen (DE-A-2 441 761). Analoges gilt für Mischungen aus Dextran und Salzen (C.A. *83*, 188 805 g (1975)). Schließlich sind Polysaccharide — wie Dextran — als Hilfsstoffe bei der Chromatographie verwendet worden (FR-A-2 259 836).

Es wurde nun ein Verfahren gefunden, das die oben genannten Nachteile vermeidet.

Gegenstand der Erfindung ist ein Verfahren zur Herstellung eines festen, abriebfesten Formkörpers mit einem Gehalt an einem Arzneimittelwirkstoff oder einem Diagnostikum, dadurch gekennzeichnet, daß man den Arzneimittelwirkstoff oder das Diagnostikum in einer mindestens 6 %igen Lösung von Dextran in Wasser löst, die Lösung in eine Form füllt und lyophilisiert.

Der erfindungsgemäß erhaltene Formkörper besitzt nicht die übliche lockere Form eines Lyophilisats, welches bei Druck sofort zerfällt, er liegt vielmehr — völlig überraschend — in fester und abriebfester Form vor. Das neue Verfahren ermöglicht es daher, auch hochempfindliche Substanzen, wie Enzyme und Proteine in eine stabile feste Form zu bringen, was bislang nur in Ausnahmefällen möglich war. Diese Formen sind darüber hinaus sehr abriebfest.

Sofern die Formkörper bei der Lyophilisation nicht zu stark getrocknet werden, lassen sie sich sehr gut pressen, ohne dabei zu zerfallen. Ein weiterer Vorteil der neuen Form liegt darin, daß zur Formgebung nur ein Hilfsstoff — nämlich Dextran — notwendig ist.

Man füllt die Lösung zum Lyophilisieren in Formen, welche die Gestalt der gewünschten Zubereitung besitzen. Solche Formen sind z. B. Zylinder oder Würfel. Nach der Lyophilisation erhält man die Mischung aus Substanz und Dextran in Form einer abriebfesten Tablette, die — falls gewünscht — noch gepreßt werden kann, wodurch die Auflösegeschwindigkeit der Tablette herabgesetzt wird.

In der neuen Zubereitung lassen sich die darin enthaltenen Substanzen sehr genau dosieren, was insbesondere für Diagnostika von großer Bedeutung ist. Besonders günstig ist es hierfür, daß auch empfindliche Substanzen bei der Formgebung praktisch nicht inaktiviert werden. Ein weiterer Vorteil besteht darin, daß die Zubereitung in Wasser sehr schnell zerfällt bzw. sich sehr schnell in Wasser löst, falls sie nicht gepreßt wurde.

Empfindliche Substanzen sind in der neuen Form wesentlich stabiler als in reinem Zustand und können daher auch bei Raumtemperatur über einen längeren Zeitraum aufbewahrt werden, ohne an Aktivität zu verlieren.

Als Dextran eignet sich für die neue Zubereitung beispielsweise ein Dextran mit dem Molekulargewicht von 20 000, 40 000 oder 60 000. Die Menge an Dextran in der zu lyophilisierenden Lösung soll wenigstens 6 % betragen.

In die Formkörper können alle Substanzen eingearbeitet werden, die in Wasser löslich oder darin suspendierbar sind. Besonders eignen sie sich für empfindliche pharmazeutische Wirkstoffe sowie Enzyme für diagnostische Zwecke und biochemische Analysen. Beispiele dafür sind Antibiotika, wie Penicilline, Streptomycine und Tetracycline und Enzyme, wie Fibrinogenase, Aminosäureoxidase, Streptokinase, Urokinase, Caseinase. Diese Substanzen können in einer Menge von 10 bis 50 % in dem lyophilisierten Produkt vorliegen.

Schließlich lassen sich auch Hilfsstoffe wie Puffer, Lösungsvermittler oder Salze in die Förmkörper einarbeiten, wodurch enzymatische Bestimmungen wesentlich vereinfacht werden.

Die Formkörper nehmen bis zu etwa 10 % Luftfeuchtigkeit auf, ohne daß sich ihre Eigenschaften dadurch verändern. Falls daher feuchtigkeitsempfindliche Substanzen in diesen Zubereitungen aufbewahrt werden sollen, müssen sie unter Ausschluß von Luftfeuchtigkeit aufbewahrt werden.

Beispiel 1

Arzneimittel

In einer 10 %igen Dextran-Lösung (MG = 40 000) werden 2 % Verapamil gelöst. Jeweils 0,5 ml der Lösung werden zu Formlingen lyophilisiert, welche 50 mg Dextran und 10 mg Verapamil enthalten. Beim Stehen an der Luft nehmen die Formlinge etwa 6 mg Wasser auf.

Beispiel 2

Diagnostikum zur Fibrinogenbestimmung

Jeweils 0,2 ml einer Mischung aus 10 ml Ancrod-Lösung (enthaltend 70 IU Fibrinogenase pro ml), 50 ml 10 %ige Dextran-Lösung (MG = 40 000), welche 0,9 % NaCl enthält, und 40 ml 0,9 %ige NaCl-Lösung werden zu Formlingen lyophilisiert. Die Formlinge enthalten 1,2 IU des Enzyms und wiegen 12 mg in trockenem Zustand.

Die gleichen Formlinge erhält man, wenn man Lösungen von Dextran mit dem Molekulargewicht 20 000 oder 60 000 verwendet.

Beispiel 3

Diagnostikum zur Fibrinogenbestimmung

10 ml Ancrod-Lösung (enthaltend 70 IU Fibrinogenase pro ml), 50 ml 10 %ige Dextran-Lösung (MG = 40 000), welche 0,9 % NaCl enthält und 40 ml Puffer werden gemischt. Jeweils 0,2 ml der Lösung werden zu Formlingen lyophilisiert.

Der Puffer bestand aus 250 ml einer wäßrigen Lösung von 1,838 g Natriumacetat und 7,36 Barbitalnatrium, 200 ml 4,25 %iger NaCl-Lösung, 217 ml 0,1 N HCl, 638 ml destilliertem Wasser. Der pH-Wert des Puffers war auf 7,4 eingestellt worden.

Zur Lyophilisation in den Beispielen 1-3 wurden die Lösungen in die Vertiefungen von Mikrotiterplatten pipettiert. Anschließend wurden die Lösungen gefroren und bei −30 °C und 0,07 mbar getrocknet.

**Ansprüche**

1. Verfahren zur Herstellung eines festen, abriebfesten Formkörpers mit einem Gehalt an einem Arzneimittelwirkstoff oder einem Diagnostikum, dadurch gekennzeichnet, daß man den Arzneimittelwirkstoff oder das Diagnostikum in einer mindestens 6 %igen Lösung von Dextran in Wasser löst, die Lösung in eine Form füllt und lyophilisiert.

2. Verfahren gemäß Anspruch 1, dadurch gekennzeichnet, daß man den Formkörper preßt.

3. Verfahren gemäß Anspruch 1, dadurch gekennzeichnet, daß man als Arzneimittelwirkstoff oder Diagnostikum ein Antibiotikum oder ein Enzym verwendet.

4. Verfahren gemäß Anspruch 1 oder 3, dadurch gekennzeichnet, daß man als Arzneimittelwirkstoff oder Diagnostikum Penicilline, Streptomycine, Tetracycline, Fibrinogenase, Aminosäureoxidase, Streptokinase, Urokinase oder Caseinase verwendet.

5. Verfahren gemäß Anspruch 1, dadurch gekennzeichnet, daß man den Arzneimittelwirkstoff oder das Diagnostikum in einer Menge von 10 bis 50 % bezogen auf das lyophilisierte Produkt einsetzt.

**Claims**

1. A process for the production of a solid, abrasion-resistant moulding containing a pharmaceutically active compound or a diagnostic agent, wherein the pharmaceutically active compound or the diagnostic agent is dissolved in an at least 6 % strength solution of dextran in water, and the solution is filled into a mould and lyophilized.

2. A process as claimed in claim 1, wherein the moulding is compressed.

3. A process as claimed in claim 1, wherein an antibiotic is used as the pharmaceutically active compound, and an enzyme is used as the diagnostic agent.

4. A process as claimed in claim 1 or 3, wherein a penicillin, streptomycin or tetracyclin is used as the pharmaceutically active compound, and fibrinogenase, aminoacid-oxidase, streptokinase, urokinase or caseinase is used as the diagnostic agent.

5. A process as claimed in claim 1, wherein the pharmaceutically active compound or diagnostic agent is used in an amount of from 10 to 50 %, based on the lyophilized product.

**Revendications**

1. Procédé de préparation d'un corps moulé solide, résistant à l'abrasion, contenant un principe actif de médicament ou un produit de diagnostic, caractérisé par le fait qu'on dissout le principe actif de médicament ou le produit de diagnostic dans une solution à au moins 6 % de dextrane dans l'eau, on remplit un moule de cette solution et on lyophilise.

2. Procédé selon la revendication 1, caractérisé par le fait qu'on presse le corps moulé.

3. Procédé selon la revendication 1, caractérisé par le fait qu'on utilise, comme principe actif de médicament, un produit de diagnostic, un antibiotique ou un enzyme.

4. Procédé selon la revendication 1 ou 3, caractérisé par le fait qu'on utilise, comme principe actif de médicament, un produit de diagnostic, de la pénicilline, streptomycine, tétracycline, fibrinogenase, oxydase d'aminoacide, streptokinase, urokinase ou caséinase.

5. Procédé selon la revendication 1, caractérisé par le fait qu'on introduit le principe actif de médicament ou le produit de diagnostic en proportion de 10 à 50 %, rapportée au produit lyophilisé.